(19) Europäisches Patentamt
European Patent Office
Office européen des brevets

(11) Veröffentlichungsnummer: **0 056 153**
**B1**

(12) # EUROPÄISCHE PATENTSCHRIFT

(45) Veröffentlichungstag der Patentschrift: **04.12.85**

(51) Int. Cl.⁴: **C 08 G 18/30**, C 08 G 18/78, C 07 D 251/54, C 07 D 251/70, C 08 G 59/32, C 08 G 18/83

(21) Anmeldenummer: **81110806.7**

(22) Anmeldetag: **29.12.81**

(54) **Verfahren zur Herstellung von s-Triazin-Einheiten und Epoxidgruppen aufweisenden Verbindungen und ihre Verwendung als einbaufähige Füllstoffe bei der Herstellung von Polyurethan-Kunststoffen.**

(30) Priorität: **08.01.81 DE 3100355**

(43) Veröffentlichungstag der Anmeldung: **21.07.82 Patentblatt 82/29**

(45) Bekanntmachung des Hinweises auf die Patenterteilung: **04.12.85 Patentblatt 85/49**

(84) Benannte Vertragsstaaten: **DE**

(56) Entgegenhaltungen:
**EP - A - 0 000 893**
**DE - A - 2 319 160**
**US - A - 3 440 230**
**US - A - 3 761 452**

**Patent Abstracts of Japan, Band 3, Nr. 79, 6. Juli 1979, Seite 19C51**

(73) Patentinhaber: **BAYER AG, Konzernverwaltung RP Patentabteilung, D-5090 Leverkusen 1 Bayerwerk (DE)**

(72) Erfinder: **Rasshofer, Werner, Dr., Wolfskaul 10, D-5000 Koeln 80 (DE)**
Erfinder: **Grögler, Gerhard, Dr., von-Diergardt-Strasse 46, D-5090 Leverkusen 1 (DE)**
Erfinder: **Kopp, Richard, Dr., Wolfskaul 12, D-5000 Koeln 80 (DE)**

**EP 0 056 153 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein Verfahren zur Herstellung neuer s-Triazin-Einheiten und Epoxidgruppen aufweisenden Verbindungen und ihre Verwendung als einbaufähige Füllstoffe für damit härtbare Isocyanatharze und zur Modifizierung von Polyurethan-Kunststoffen.

In der EP-A-0 000 893 werden s-Triazin-Einheiten aufweisende Polyisocyanate beschrieben, die durch Umsetzung von Melamin mit überschüssigen Mengen an aromatischen Diisocyanaten mit Isocyanatgruppen einer unterschiedlichen Reaktivität erhalten werden. Diese in der Vorveröffentlichung beschriebenen Verbindungen stellen in organischen Medien schwer- bis unlösliche Festkörper dar, die sehr hochschmelzend sind. Wegen ihres hohen Schmelzpunktes und ihrer schlechten Löslichkeit bzw. Verträglichkeit sind der Verwendbarkeit der genannten Polyisocyanate als reaktive Füllstoffe bei der Herstellung von Polyurethanen nach dem Isocyanat-Polyadditionsverfahren enge Grenzen gesetzt.

Es ist daher die Aufgabe der vorliegenden Erfindung, ausgehend von den bekannten s-Triazin-Einheiten aufweisenden Polyisocyanaten einbaufähige Füllstoffe zur Verfügung zu stellen, die nicht mit den genannten Nachteilen behaftet sind, d. h. die wesentlich tiefer (<300° C) als obige s-Triazin-Einheiten aufweisende Isocyanate schmelzen und/oder die eine verbesserte Verträglichkeit mit den zur Herstellung von Polyurethan-Kunststoffen eingesetzten Ausgangsmaterialien aufweisen. Diese Aufgabe konnte durch das nachstehend näher beschriebene, erfindungsgemäße Verfahren gelöst werden.

Gegenstand der Erfindung ist somit ein Verfahren zur Herstellung von s-Triazin-Einheiten und Epoxid-Gruppen aufweisenden Verbindungen, dadurch gekennzeichnet, daß man Triisocyanate der Formel

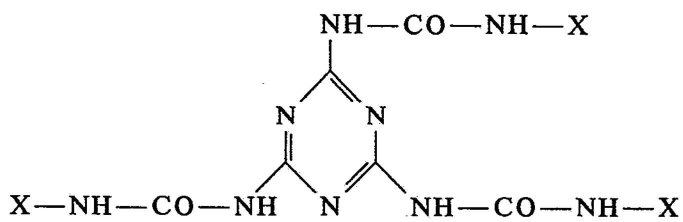

welche durch Umsetzung von Melamin mit Diisocyanaten der Formel X—NCO unter Bildung von Harnstoffgruppen hergestellt worden sind, wobei

X für einen Rest steht, wie er duch Entfernung der höherreaktiven Isocyanatgruppe aus einem aromatischen Diisocyanat mit Isocyanatgruppen unterschiedlicher Reaktivität im Sinne der Isocyanat-Additionsreaktion erhalten wird,

mit NCO-reaktive Gruppen enthaltenden Epoxid-Verbindungen unter Einhaltung eines Äquivalentverhältnisses zwischen Isocyanatgruppen und NCO-reaktiven Gruppen von $\leq 1:1$ zur Reaktion bringt.

Gegenstand der Erfindung ist auch die Verwendung der erfindungsgemäßen Verfahrensprodukte als Füllstoffkomponente bei der Herstellung von Polyurethankunststoffen.

Beim erfindungsgemäßen Verfahren werden s-Triazin-Einheiten aufweisende Polyisocyanate der obengenannten Formel eingesetzt, wobei X die obengenannte Bedeutung hat. Vorzugsweise kommen solche Polyisocyanate der genannten Formel zum Einsatz, für welche X für einen 3-Isocyanato-4-methylphenyl- oder einen 4-(2-Isocyanatobenzyl)-phenyl-Rest steht. Die Herstellung derartiger Polyisocyanate ist beispielsweise in der EP-A-0 000 893 beschrieben.

Für das erfindungsgemäße Verfahren geeignete s-Triazin-Einheiten aufweisende Polyisocyanate sind demzufolge insbesondere N,N',N''-Tri-(3-isocyanato-4-methylphenyl-aminocarbonyl)-melamin oder N,N',N''-Tri-[4-(2-isocyanatobenzyl)-phenyl-aminocarbonyl]-melamin. Es können jedoch beim erfindungsgemäßen Verfahren auch beliebige andere, obiger Definition entsprechende s-Triazin-Einheiten aufweisende aromatische Polyisocyanate eingesetzt werden.

Reaktionspartner für die genannten s-Triazin-Einheiten aufweisende Polyisocyanate sind beliebige Epoxid-Verbindungen, die außer Epoxidgruppen noch mindestens eine im Sinne der Isocyanat-Additionsreaktion reaktive Gruppe und ein Molekulargewicht von 74—1000, insbesondere 74—500, aufweisen. Bevorzugte Epoxid-Verbindungen sind solche der genannten Definition, die eine Epoxid- und eine gegenüber Isocyanatgruppen reaktionsfähige, vorzugsweise alkoholische Hydroxylgruppe aufweisen.

Geeignete Reaktionspartner für die s-Triazin-Einheiten aufweisenden Polyisocyanate sind insbesondere:

a) 2,3-Epoxy-1-alkanole wie Glycidol (2,3-Epoxy-1-propanol) und seine niedermolekularen Kondensationsprodukte mit sich selbst, weitere 2,3-Epoxyalkohole wie z. B. 2,3-Epoxy-1-butanol, 2,3-Epoxy-1-pentanol, 2,3-Epoxy-2-methyl-1-butanol sowie deren niedermolekulare Kondensationsprodukte mit sich selbst;
b) Glycidylester von Hydroxycarbonsäuren wie z. B. Milchsäure, Glykolsäure, Hydroxypivalinsäure,

4-(2-Hydroxyethoxy)-benzoesäure, 3-Chlor-4-(2-hydroxyethoxy)-benzoesäure;

c) Monoglycidylether mehrwertiger Alkohole wie Alkandiole oder Alkendiole, z. B. Ethylenglykol, 1,4-Dihydroxybutan, Di-, Tri- und Polyethylenglykole (Molmasse bis ca. 800), Glycerin, Pentaerythrit und Mannit. Monoglycidylether mehrwertiger Phenole wie z. B. Hydrochinon, Resorcin, Bisphenol-A, Bisphenol F, können gleichfalls verwendet werden, sind aber weniger bevorzugt.

Bevorzugt sind 2,3-Epoxy-1-alkanole, besonders bevorzugt ist Glycidol.

Bei der Durchführung des erfindungsgemäßen Verfahrens werden die Komponenten in solchen Mengenverhältnissen eingesetzt, daß lediglich solche Verfahrensprodukte entstehen, die keine freien NCO-Gruppen mehr besitzen. Dies bedeutet, daß beim erfindungsgemäßen Verfahren die s-Triazin-Einheiten enthaltenden Polyisocyanate und die NCO-reaktive Gruppen enthaltenden Epoxid-Verbindungen in einem Verhältnis der NCO-reaktiven Gruppen zu den NCO-Gruppen von 1 : 1 bis 10 : 1, bevorzugt 1,1 : 1 bis 5 : 1, zur Umsetzung gebracht werden. Da es sich bei den Epoxidgruppen aufweisenden Reaktionspartnern für die s-Triazin-Einheiten aufweisenden Polyisocyanaten um z. T. destillierbare und/oder um im allgemeinen in gängigen Lösungsmitteln wie Diethylether oder Aceton leicht lösliche Verbindungen handelt, kann nach der Durchführung der erfindungsgemäßen Umsetzung der gegebenenfalls vorliegende Überschuß an nicht umgesetzten Epoxidgruppen aufweisenden Ausgangsverbindungen leicht durch Destillation und/oder Extraktion und/oder Filtration von den erfindungsgemäßen Verfahrensprodukten entfernt werden. Bei der erfindungsgemäßen Umsetzung wird die Art der Reaktionspartner im allgemeinen so gewählt, daß Verfahrensprodukte mit einem maximalen Molekulargewicht von ca. 4000, vorzugsweise 2500, entstehen. Bei der erfindungsgemäß bevorzugten Umsetzung von s-Triazin-Einheiten aufweisenden Triisocyanaten der obengenannten Art mit eine Epoxidgruppe und eine Hydroxylgruppe aufweisenden Reaktionspartnern entstehen beim erfindungsgemäßen Verfahren als bevorzugte Verfahrensprodukte die der erfindungsgemäßen Reaktion entsprechenden trifunktionellen Epoxide, deren Molekulargewicht aus ihrem Gehalt an Epoxidgruppen errechnet werden kann.

Die Durchführung des erfindungsgemäßen Verfahrens kann beispielsweise dergestalt erfolgen, daß man eine Mischung aus einem Polyisocyanat der oben beispielhaft genannten Art und mindestens einer, Epoxidgruppen und gegenüber Isocyanatgruppen reaktionsfähige Gruppen aufweisenden, Verbindung der oben beispielhaft genannten Art während eines Zeitraums von 2—100 Stunden, vorzugsweise 4—25 Stunden, unter Rühren zur Reaktion bringt. Das erfindungsgemäße Verfahren wird im allgemeinen im Temperaturbereich zwischen 0° und 100°C, vorzugsweise zwischen 20° und 80°C, durchgeführt. Die Umsetzung kann in Substanz oder in einem gegenüber Isocyanatgruppen inerten Lösungsmittel wie z. B. Dioxan, Tetrahydrofuran, Benzol, Toluol, Chlorbenzol, Dichlorbenzol, Nitrobenzol, Xylol, chlorierten aliphatischen Kohlenwasserstoffen oder dipolar aprotischen Solventien wie N,N-Dimethylformamid, Dimethylsulfoxid, Hexamethylphosphorsäuretriamid oder Sulfolan durchgeführt werden. Bevorzugtes Lösungsmittel ist Dimethylformamid.

Beim erfindungsgemäßen Verfahren werden oft die an sich bekannten Beschleuniger für die Isocyanat-Additionsreaktion mitverwandt. Hierzu gehören beispielsweise Amidine wie Diazabicycloundecen, N-Methyl-2-methyltetrahydropyrimidin, tert. Amine wie Triethylendiamin (DABCO®) oder Metall-Katalysatoren wie Sn-(II)-octoat, Dibutylzinndilaurat oder Bleioctoat.

Beim erfindungsgemäßen Verfahren entstehen als Verfahrensprodukte Verbindungen, die im allgemeinen einen Gehalt an s-Triazin-Einheiten von ca. 2,1—9 Gew.-%, vorzugsweise 4,2—9 Gew.-%, und einen Gehalt an Epoxidsauerstoff von ca. 1,2—5,5 Gew.-%, vorzugsweise 1,9—5,5 Gew.-%, aufweisen. Da die erfindungsgemäßen Verfahrensprodukte insbesondere bei Raumtemperatur nur eine begrenzte Löslichkeit in organischen Lösungsmitteln aufweisen, können sie durch Kristallisation und anschließendes Waschen mit Extraktionsmitteln wie z. B. Diethylether oder Aceton vor überschüssigen Mengen an Epoxidgruppen aufweisenden Ausgangsverbindungen befreit werden.

Je nach Art der eingesetzten Ausgangsmaterialien entstehen beim erfindungsgemäßen Verfahren Verfahrensprodukte eines breiten Schmelzpunktbereichs. Die erfindungsgemäßen Verfahrensprodukte unterscheiden sich von den s-Triazin-Einheiten aufweisenden Ausgangspolyisocyanaten durch einen verminderten Schmelzpunkt und/oder eine verbesserte Verträglichkeit mit den bei der Herstellung von Polyurethankunststoffen üblicherweise eingesetzten Ausgangsmaterialien.

Die erfindungsgemäßen Verfahrensprodukte eignen sich besonders als anteilige Reaktiv-Vernetzerkomponenten bei der Herstellung von Polyurethankunststoffen, wobei sie bei dieser erfindungsgemäßen Verwendung der Komponente mit gegenüber Isocyanatgruppen reaktionsfähigen Gruppen einverleibt werden. Es ist auch möglich, die erfindungsgemäßen Verfahrensprodukte als alleinige NCO-reaktive Komponente mit organischen Polyisocyanaten umzusetzen. Die erfindungsgemäßen Verfahrensprodukte sind schließlich auch als Vernetzer für Polyurethankunststoffe und andere Polymere, wie sie bei der Herstellung von Lacken, Klebstoffen, Elastomeren oder Schaumstoffen Verwendung finden, geeignet. Sie eignen sich schließlich auch als Vernetzerkomponente bei der Herstellung von Polyepoxiden.

Die in den nachstehenden Beispielen gemachten Prozentangaben beziehen sich auf Gew.-%.

Beispiel 1

Umsetzung von N,N',N''-Tris-(3-isocyanato-4-methylphenyl-aminocarbonyl)-melamin mit 2,3-Epoxypropanol

148 g N,N',N''-Tris-(3-isocyanato-4-methylphenyl-aminocarbonyl)-melamin (0,23 mol), 60 g 2,3-Epoxypropanol (0,81 mol) und 0,5 g Zinn-2-ethylhexanoat werden in 200 ml Dimethylformamid suspendiert resp. gelöst. Es wird 15 h bei 60° C gerührt und danach die auf Raumtemperatur abgekühlte Reaktionsmischung abgesaugt und mit 150 ml Diethylether gewaschen. Es werden nach Trocknen bei 15 Torr/50° C bis zur Gewichtskonstanz 198 g einer farblosen, bei 240° C schmelzenden Verbindung, die einen Gehalt an s-Triazin-Gruppen von 9% aufweist, erhalten. Der Gehalt an Epoxidsauerstoff liegt bei 5,3% (Theorie: 5,5%).

Beispiel 2

Umsetzung von N,N',N''-Tris-(3-isocyanato-4-methylphenyl-aminocarbonyl)-melamin mit Glycerin-1-glycidylether

207 g N,N',N''-Tris-(3-isocyanato-4-methylphenyl-aminocarbonyl)-melamin (0,32 mol), 148 g Glycerin-1-glycidylether (1 mol) und 0,75 g Zinn(II)-(2-ethyl-hexanoat) werden in 200 ml Dimethylformamid ansuspendiert resp. gelöst. Es wird 25 h bei 60° C gerührt und danach die auf Raumtemperatur abgekühlte Reaktionsmischung abgesaugt und mit 150 ml Aceton gewaschen. Es werden nach Trocknen bei 15 Torr/50° C bis zur Gewichtskonstanz 347 g einer farblosen, bei 270° C schmelzenden Verbindung, die einen Gehalt an s-Triazin-Gruppen von 7,1% aufweist, erhalten. Der Gehalt an Epoxidsauerstoff liegt bei 4,3% (Theorie: 4,4%).

Beispiel 3

50 g eines NCO-Prepolymers aus Toluylen-2,4-diisocyanat und einem Polypropylenetherglykol des Molekulargewichts 2000, das einen Isocyanatgehalt von 3,5% aufweist, werden mit 12,5 g des fein gemahlenen Epoxids aus Beispiel 1 und 0,5 ml Bortrifluorid-Etherat intensiv vermischt und 3 h bei 130° C zu einem weichen, klebfreien Kunststoff ausgehärtet. Ein gleiches Ergebnis wird erhalten, wenn das Gemisch während 3 Tagen bei Raumtemperatur gehärtet wird.

Beispiel 4

50 g eines NCO-Prepolymers aus Toluylen-2,4-diisocyanat und einem trifunktionellen, auf Trimethylolpropan gestarteten PO/EO-Polyetherpolyol des Molekulargewichts 6000, das einen Isocyanatgehalt von 2,67% aufweist, werden mit 8 g des fein vermahlenen Epoxids aus Beispiel 1 und 0,5 ml Bortrifluorid-Etherat intensiv vermischt und in einer offenen Metallform 3 h bei 130° C zu einem halbharten, klebfreien und elastischen Kunststoff ausgehärtet.

Beispiel 5

50 g des fein vermahlenen Epoxids aus Beispiel 1 (57,5 mmol) werden mit 43,5 g eines Semiprepolymers mit 18,3 Gew.-% NCO, welches hergestellt wurde durch Umsetzung eines Polyisocyanates, das durch Phosgenierung von Anilin-Formaldehyd-Kondensat erhalten wurde und eine Viskosität von 130 mPa · s (25° C) und einen NCO-Gehalt von 31 Gew.-% aufweist, mit einem Polyether der OH-Zahl 42, der durch Anlagerung eines Gemisches von Propylenoxid und Ethylenoxid an ein Gemisch aus Trimethylolpropan und Propylenglykol (Molverhältnis 3 : 1) erhalten wurde, und 2,5 ml Bortrifluorid-Etherat intensiv vermischt und in eine offene Metallform mit einem Durchmesser von 10 cm gegeben. Die Masse härtet bei 130° C innerhalb 2 h zu einem harten, klebfreien Kunststoff aus.

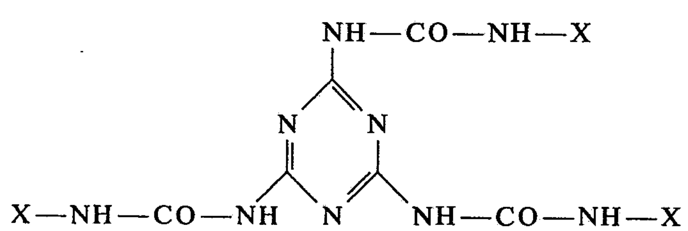

**Patentansprüche**

1. Verfahren zur Herstellung von s-Triazin-Einheiten und Epoxidgruppen aufweisenden Verbindungen, dadurch gekennzeichnet, daß man Triisocyanate der Formel

welche durch Umsetzung von Melamin mit Diisocyanaten der Formel X—NCO unter Bildung von Harnstoffgruppen hergestellt worden sind, wobei

X für einen Rest steht, wie er durch Entfernung der höher-reaktiven Isocyanatgruppe aus einem aromatischen Diisocyanat mit Isocyanatgruppen unerschiedlicher Reaktivität im Sinne der Isocyanat-Additionsreaktion erhalten wird,

mit NCO-reaktive Gruppen enthaltenden Epoxid-Verbindungen unter Einhaltung eines Äquivalentverhältnisses zwischen Isocyanatgruppen und NCO-reaktiven Gruppen von ≤1 : 1 zur Reaktion bringt.

2. Verfahren gemäß Anspruch 1, dadurch gekennzeichnet, daß man als NCO-reaktive Gruppen enthaltende Epoxidverbindungen 2,3-Epoxy-1-alkanole verwendet.

3. Verfahren gemäß Ansprüchen 1 und 2, dadurch gekennzeichnet, daß man als Verbindungen der in Anspruch 1 genannten Formel solche verwendet, für welche

X für einen 3-Isocyanato-4-methylphenyl- oder einen 4-(2-Isocyanatobenzyl)-phenylrest

steht.

4. Verwendung der gemäß Ansprüchen 1—3 erhältlichen Verbindungen als Füllstoffkomponente bei der Herstellung von Polyurethankunststoffen.

**Claims**

1. Process for the production of compounds containing s-triazine units and epoxide groups, characterised in that triisocyanates of the formula

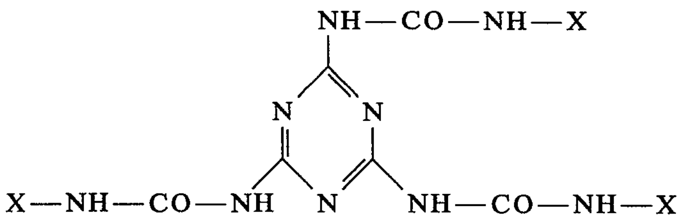

which have been produced by reacting melamine with diisocyanates of the formula X—NCO, with the formation of urea groups, wherein

X represents a radical of type obtained by removing the more highly reactive isocyanate group from an aromatic diisocyanate containing isocyanate groups of differing reactivity with regard to the isocyanate addition reaction,

are reacted with epoxide compounds containing NCO-reactive groups, while maintaining an equivalent ratio between isocyanate groups and NCO-reactive groups of ≤1 : 1.

2. Process according to Claim 1, characterised in that 2,3-epoxy-1-alkanols are used as the epoxide compounds containing NCO-reactive groups.

3. Process according to Claims 1 and 2, characterised in that those compounds for which

X represents a 3-isocyanato-4-methylphenyl or 4-(2-isocyanatobenzyl)-phenyl radical

are used as the compounds of the formula mentioned in Claim 1.

4. Use of the compounds obtainable according to Claim 1—3 as a filler component in the production of polyurethane plastics.

**Revendications**

1. Procédé de préparation de composés à motifs s-triazine et à groupes époxydes, caractérisé en ce qu'on fait réagir des triisocyanates de formule

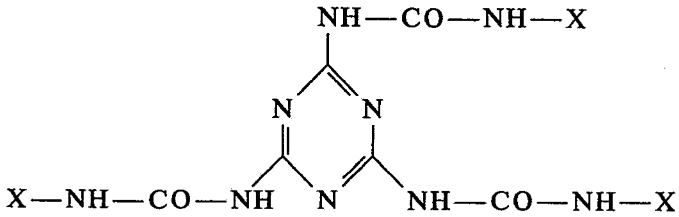

préparés par réaction de mélamine avec des diisocyanates de formule X—NCO avec formation de groupes urée, où

X représente un résidu obtenu par séparation du groupe isocyanate hautement réactif d'un diisocyanate aromatique possédant des groupes isocyanates de réactivités différentes au sens de la réaction d'addition des isocyanates,

avec des composés époxydes à groupes réactifs vis-à-vis de NCO en adoptant un rapport d'équivalents entre les groupes isocyanates et les groupes réactifs vis-à-vis de NCO inférieur ou égal à 1 : 1.

2. Procédé selon la revendication 1, caractérisé en ce qu'on emploie comme composés époxydes à groupes réactifs vis-à-vis de NCO, des 2,3-époxy-1-alcanols.

3. Procédé selon les revendications 1 et 2, caractérisé en ce qu'on emploi comme composés de formule selon la revendication 1 ceux dans lesquels

X représente un radical 3-isocyanato-4-méthylphényle ou 4-(2-isocyanatobenzyl)-phényle.

4. L'utilisation des composés obtenus selon les revendications 1 à 3 comme substances de charge dans la préparation de matières plastiques de polyuréthannes.